# FASCICULE DE BREVET EUROPEEN

(11) **EP 0 836 858 B1**
(45) Date de publication et mention de la délivrance du brevet: **02.05.2003**
(21) Numéro de dépôt: 97402205.5
(22) Date de dépôt: 23.09.1997
(51) Int. Cl.: A61M 1/00

(54) **Pièce à main pour dispositif chirurgical d'aspirations-lavages**
Handstück für chirurgische Vorrichtung zum Saugen und Spülen
Handpiece for a surgical aspiration and lavage device

(30) Priorité: 18.10.1996 FR 9612686
(43) Date de publication de la demande: 22.04.1998
(73) Titulaire: PORGES, 92350 Le Plessis-Robinson (FR)
(72) Inventeur: Marie, Frédéric, 24250 Grolejac (FR)
(74) Mandataire: Bonnetat, Christian

(56) Documents cités:
- EP-A- 0 684 015
- DE-A- 1 491 710
- US-A- 5 447 494
- US-A- 5 470 305

## Description

La présente invention a pour objet un dispositif chirurgical pour réaliser des aspirations et des lavages de sites opératoires ou de cavités corporelles.

Bien que non exclusivement, le dispositif de la présente invention est particulièrement approprié à être utilisé en chirurgie coelioscopique et il est décrit ci-après principalement en relation avec cette utilisation.

On sait que la chirurgie coelioscopique ne permet pas de nettoyer un site opératoire comme cela se fait en chirurgie ouverte, par utilisation de compresses stériles par exemple. On a donc été amené à concevoir des dispositifs permettant, d'une part, d'envoyer du sérum physiologique, éventuellement chauffé, sur un site opératoire et, d'autre part, d'évacuer de ce dernier le sang, les sécrétions et le sérum physiologique souillé.

Un tel dispositif connu comporte :
- un corps oblong destiné à servir de poignée pour le chirurgien ;
- un premier raccord tubulaire, disposé à une extrémité dudit corps oblong et destiné à être mis en communication avec le site opératoire, généralement par l'intermédiaire d'une canule allongée ;
- un deuxième et un troisième raccords tubulaires, disposés à l'autre extrémité dudit corps oblong et destinés à être reliés respectivement, généralement par l'intermédiaire de conduites souples, à une source de liquide de lavage et à des moyens d'aspiration, ledit liquide de lavage provenant d'un réservoir sous pression ou bien étant acheminé sur le site opératoire uniquement par l'action de la gravité ;
- deux lignes de liaison internes audit corps oblong et reliant ledit premier raccord tubulaire respectivement auxdits deuxième et troisième raccords tubulaires, lesdites lignes de liaison pouvant être des tubes souples écrasables ; et
- des moyens de commande sélective, montés dans ledit corps oblong et susceptibles de commander la circulation fluide dans lesdites lignes de liaison, lesdits moyens de commande sélective étant actionnés par des organes d'actionnement manuels apparaissant à la surface dudit corps oblong pour être à la disposition du chirurgien. Pour chaque ligne de liaison, lesdits moyens de commande sélective peuvent comprendre un système à guillotine susceptible d'écraser le tube souple correspondant et lesdits organes d'actionnement peuvent comporter un bouton-poussoir. Généralement, en l'absence d'action du chirurgien sur le bouton-poussoir, chaque système à guillotine écrase le tube souple correspondant grâce à l'action d'au moins un ressort, ce qui interrompt la communication fluide de la ligne de liaison correspondante, alors que pour établir cette communication, le chirurgien doit appuyer sur ledit bouton-poussoir pour contrecarrer l'action dudit ressort.

En opération, le chirurgien tient ledit corps oblong dans le berceau formé par l'auriculaire, l'annulaire, le médius et l'index d'une de ses mains et actionne lesdits boutons-poussoirs avec le pouce de ladite main.

Lorsque le chirurgien appuie avec son pouce sur le bouton-poussoir associé à la ligne de liaison reliant le premier raccord tubulaire audit deuxième raccord tubulaire, sans toucher à l'autre bouton-poussoir, il établit la liaison entre ladite source de liquide de lavage et le site opératoire, tandis que la communication est coupée entre ce dernier et les moyens d'aspiration. En revanche, lorsque le chirurgien appuie avec son pouce sur le bouton-poussoir associé à la ligne de liaison reliant le premier raccord tubulaire audit troisième raccord tubulaire, sans toucher à l'autre bouton-poussoir, il établit la liaison entre le site opératoire et lesdits moyens d'aspiration, tandis que la communication est coupée entre ladite source de liquide de lavage et ledit site opératoire.

US 5 470 305 décrit un dispositif chirurgical selon le préambule de la revendication 1.

Bien que donnant toute satisfaction opérationnellement, de tels dispositifs connus ne sont pas toujours d'une grande commodité d'utilisation.

En effet, de façon usuelle, ledit premier raccord tubulaire est généralement prolongé par une canule allongée pouvant avoir une longueur de plusieurs dizaines de centimètres, l'extrémité libre de ladite canule devant déboucher sur le site ou dans la cavité concernés. Du fait que la main du chirurgien se trouve au-dessus du patient allongé sur la table d'opération et que ladite canule allongée, de longueur importante, se trouve en prolongement dudit corps oblong, le chirurgien est obligé de basculer la main d'avant en arrière autour de son poignet pour que ladite canule allongée prenne une position inclinée telle que son extrémité libre débouche à l'endroit désiré. Il en résulte une position manuelle inconfortable et fatigante pour le chirurgien.

Par ailleurs, la prise usuelle décrite ci-dessus peut ne pas convenir à certains chirurgiens qui préféreraient une prise différente, bien entendu compatible avec la disposition et la souplesse des conduites reliant le dispositif à la source de liquide de lavage et aux moyens d'aspiration.

La présente invention a pour objet principal d'accroître la commodité d'utilisation des dispositifs permettant de réaliser des aspirations et des lavages de sites et cavités corporels.

A cette fin, le dispositif selon l'invention est décrit dans la revendication 1.

Ainsi, grâce à la forme coudée dudit corps oblong et à l'interchangeabilité desdits premier et second embouts aux extrémités de celui-ci, il est possible de modifier la structure du dispositif de l'invention pour éliminer les inconvénients des dispositifs connus. Par exemple, on conçoit aisément que, lorsque ledit premier embout est monté sur l'extrémité coudée dudit corps oblong, la canule allongée montée sur ledit premier raccord tubulaire prend naturellement la position inclinée appropriée, sans que le chirurgien ait besoin de basculer son poignet d'avant en arrière. Par ailleurs, la présente invention permet d'augmenter les possibilités de prises dudit dispositif par la main d'un chirurgien, pour que chacun ait une prise qui lui soit confortable. Par exemple, ledit second embout est monté sur ladite extrémité coudée dudit corps oblong alors que ledit premier embout est monté à l'autre extrémité de celui-ci, le chirurgien peut tenir ledit corps oblong avec ledit second embout dirigé vers le haut, ses doigts autres que le pouce étant disposés du côté desdits boutons-poussoirs pour pouvoir les actionner.

Les deux parties dudit corps oblong, de longueurs inégales et séparées par ledit coude, peuvent être au moins approximativement rectilignes.

De préférence, l'angle compris entre les axes desdites parties du corps oblong est au moins égal à 90°. Cet angle peut être adapté au confort du chirurgien. On peut, par exemple à cet effet, fabriquer des corps oblongs avec des coudes plus ou moins prononcés, chaque chirurgien choisissant alors le corps oblong dont la courbure lui convient le mieux. Cependant, généralement, l'angle précité est au moins approximativement égal à 120°.

Dans un mode de réalisation avantageux, ledit corps oblong comporte, à chacune de ses extrémités, une jupe entourant les deux passages traversants correspondants, lesdites jupes étant identiques et saillant vers l'extérieur, et chacun desdits premier et second embouts comporte également une jupe entourant respectivement ledit premier et lesdits deuxième et troisième raccords tubulaires et susceptible de s'emboîter sur l'une desdites jupes dudit corps oblong.

Il est alors avantageux, pour solidariser lesdits embouts sur ledit corps oblong, que les jupes de ce dernier et celles desdits premier et second embouts comportent des moyens de verrouillage coopérants, par exemple du type à encliquetage indémontable.

Par ailleurs, ledit second embout peut comporter des prolongements respectifs desdits deuxième et troisième raccords tubulaires, en saillie par rapport à la jupe correspondante, lesdits prolongements étant susceptibles d'être introduits respectivement dans les deux passages traversants d'une extrémité dudit corps oblong.

Les deux lignes de liaison internes audit corps oblong peuvent être constituées par des conduites souples écrasables, lesdits moyens de commande sélective comprenant alors deux guillotines respectivement associées auxdites conduites souples et pressées par des moyens élastiques, tandis que lesdits organes d'actionnement sont des boutons-poussoirs solidaires desdites guillotines.

Dans un mode de réalisation particulièrement avantageux, ledit corps oblong comporte deux coques raccordées le long d'un plan de joint coudé et deux pièces d'extrémité identiques, pourvues desdits passages traversants et emprisonnées entre lesdites coques. Lesdites coques peuvent être solidarisées l'une de l'autre par encliquetage, collage ou par tout autre moyen approprié.

De plus, lesdites coques, lesdites pièces d'extrémité et lesdits premier et second embouts peuvent être réalisés en matière synthétique.

On obtient alors un dispositif facile à construire et peu coûteux, qui peut être jeté après un usage unique.

On élimine alors tous les problèmes de stérilisation après usage des appareils chirurgicaux durables.

A chaque corps oblong peuvent être associés au moins deux premiers embouts interchangeables, pourvus de canules allongées de diamètres et/ou de longueurs différents. Le chirurgien peut ainsi choisir celui des deux premiers éléments qui lui convient le mieux pour l'intervention en cours.

Les figures du dessin annexé feront bien comprendre comment l'invention peut être réalisée. Sur ces figures, des références identiques désignent des éléments semblables.
La figure 1 est une vue en perspective éclatée du corps oblong d'un dispositif chirurgical d'aspirations/lavages conforme à la présente invention.
La figure 2 est une vue en perspective d'une pièce se trouvant à chacune des extrémités du corps oblong.
La figure 3 est une vue en perspective de l'intérieur d'un premier embout d'extrémité pour le dispositif de l'invention.
La figure 4 est une vue en perspective de l'intérieur d'un second embout d'extrémité pour ledit dispositif de l'invention.
La figure 5 est une vue en coupe longitudinale du dispositif de l'invention, selon la ligne V-V de la figure 6.
La figure 6 est une vue de dessus, avec arrachement, du dispositif de l'invention.
La figure 7 est une vue latérale partielle avec arrachement (correspondant à la figure 5) illustrant l'interruption de la liaison fluide entre les deux extrémités du dispositif conforme à la présente invention.
La figure 8 est une coupe transversale selon la ligne VIII-VIII de la figure 7.
La figure 9 est une vue latérale partielle avec arrachement, semblable à la figure 7, illustrant l'établissement de la liaison fluide entre les deux extrémités du dispositif conforme à la présente invention.
La figure 10 est une coupe transversale selon la ligne X-X de la figure 9.
Les figures 11 et 12 illustrent en perspective deux possibilités de constitutions et de préhensions du dispositif conforme à la présente invention.
Les figures 13 et 14 illustrent schématiquement le fonctionnement du dispositif conforme à l'invention.

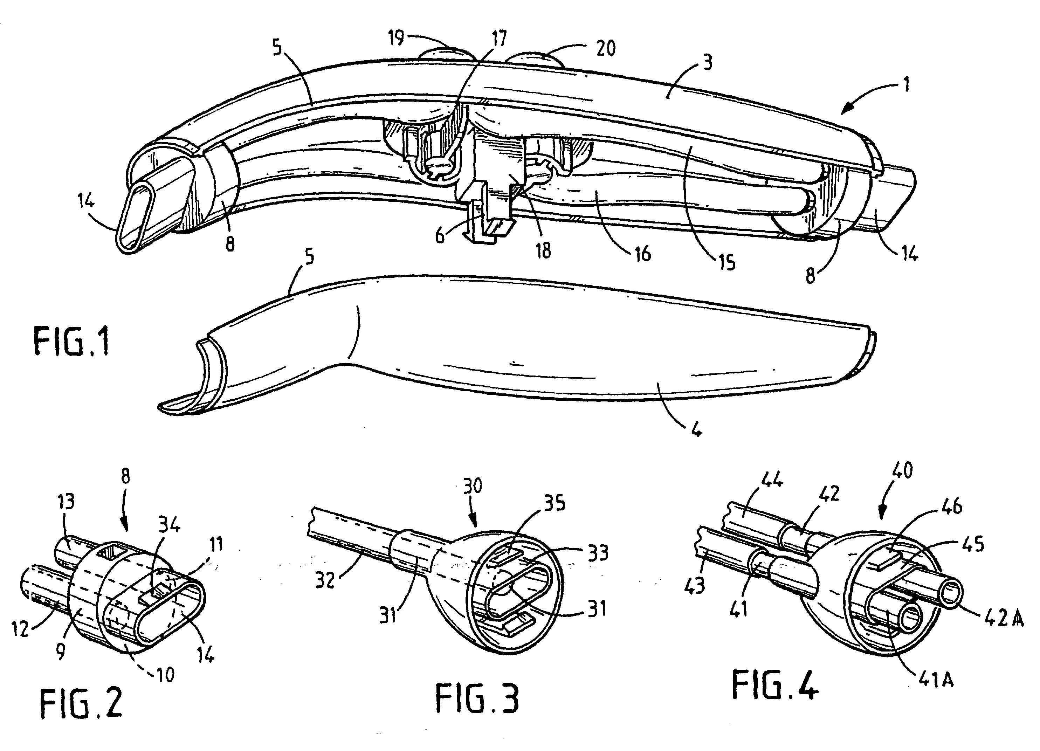

Le dispositif chirurgical, représenté sur les figures 1 à 10 et destiné à réaliser des aspirations et des lavages de sites corporels, tels que des sites opératoires, des cavités corporelles, etc ..., comporte une poignée formée par un corps oblong 1 présentant un coude 2 au voisinage de l'une de ses extrémités. Le corps oblong 1 représenté sur ces figures comporte deux parties rectilignes 1A et 1B, d'axes respectifs A-A et B-B, reliées par ledit coude 2. L'angle a entre les axes A-A et B-B est au moins égal à 90° et est généralement de l'ordre de 120°, comme représenté sur les figures.

Dans l'exemple de réalisation représenté, le corps oblong 1 comporte deux coques longitudinales 3 et 4 coudées, raccordées le long d'un plan de joint coudé longitudinal 5. Lesdites coques 3 et 4 sont solidarisées l'une de l'autre grâce à des moyens d'encliquetage coopérants 6 et 7, respectivement portés par lesdites coques.

A chacune des extrémités du corps oblong 1, une pièce d'extrémité 8 (identique pour les deux extrémités) est emprisonnée entre lesdites coques 3 et 4. Comme le montre plus particulièrement la figure 2, chaque pièce 8 comporte un corps 9, traversé par deux passages traversants 10 et 11. Du côté intérieur du corps oblong 1, ladite pièce 8 comporte deux éléments tubulaires 12 et 13, prolongeant lesdits passages traversants 10 et 11. Du côté extérieur du corps oblong, la pièce 8 comporte une jupe 14 entourant lesdits passages traversants 10 et 11.

A l'intérieur du corps oblong 1, sont prévues deux conduites longitudinales 15 et 16, souples et écrasables, chacune desdites conduites 15, 16 étant emmanchée, à l'une de ses extrémités sur le prolongement tubulaire 12 d'une desdites pièces d'extrémité 8, et à son autre extrémité sur le prolongement tubulaire 13 de l'autre desdites pièces d'extrémité 8. Ainsi, chacune desdites conduites 15, 16 relie un passage traversant 10, 11 d'une extrémité dudit corps oblong 1 à un passage traversant 11, 10 de l'autre extrémité dudit corps oblong.

Par ailleurs, sur la partie 1A du corps oblong 1, sont prévus des moyens de commande sélective susceptibles de commander la circulation fluide dans lesdites conduites souples 15, 16 et des organes d'actionnement desdits moyens de commande. Ceux-ci sont constitués par des guillotines 17, 18 respectivement associées aux conduites 15 et 16 et solidaires de boutons-poussoirs 19 et 20. Les boutons-poussoirs 19 et 20 apparaissent à l'extérieur du corps oblong 1, du côté opposé à la concavité du coude 2 et sont guidés en déplacement, orthogonalement à l'axe A-A de la partie de corps 1A. Ils sont respectivement chargés par des ressorts 21 et 22.

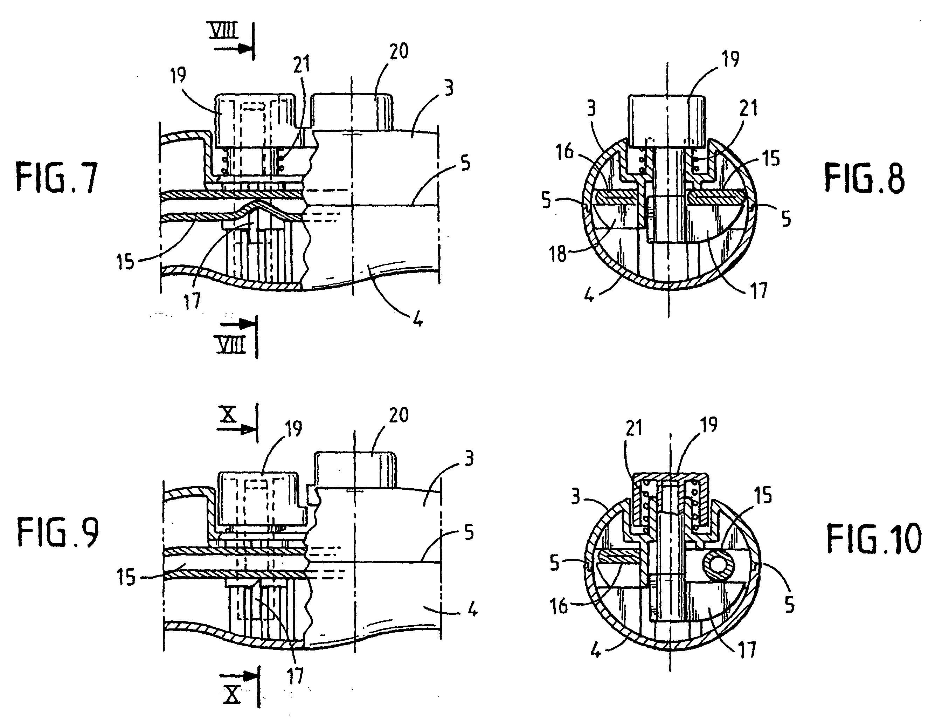

Au repos (voir les figures 7 et 8), lesdits ressorts 21 et 22 pressent lesdits boutons-poussoirs 19, 20 pour que ceux-ci saillent à l'extérieur du corps oblong 1 et pour que lesdites guillotines 17 et 18 écrasent lesdites conduites souples 15 et 16, en interdisant les communications fluides entre les passages traversants 10 et 11 des deux pièces d'extrémité 8. Lorsque l'on désire établir l'une desdites communications fluides (voir les figures 9 et 10), on enfonce le bouton-poussoir 19, 20 correspondant, à l'encontre de l'action du ressort 21, 22 associé, ce qui écarte la guillotine correspondante 17, 18 de la conduite souple 15, 16 concernée, qui reprend sa forme et permet ladite communication fluide.

En plus du corps oblong 1, le dispositif chirurgical conforme à la présente invention comporte de plus au moins un embout 30 (figure 3) et un embout 40 (figure 4).

L'embout 30 comporte un raccord tubulaire 31, dans lequel peut par exemple s'emboîter d'un côté une canule allongée 32. De l'autre côté, ledit raccord tubulaire 31 est entouré par une jupe 33, pouvant s'emboîter sur la jupe 14 de l'une quelconque des deux pièces d'extrémité 8. Lorsque la jupe 33 est emboîtée sur une jupe 14, ledit raccord tubulaire 31 est raccordé, en commun et de façon étanche, aux deux passages traversants 10 et 11 de la pièce 8 correspondante. L'embout 30 et les pièces 8 peuvent comporter des moyens de solidarisation par encliquetage coopérants, 34 et 35.

L'embout 40 comporte deux raccords tubulaires 41 et 42, destinés à être reliés, respectivement, à une source de liquide de lavage et à des moyens d'aspiration par des conduites 43 et 44. Du côté opposé aux conduites 43 et 44, lesdits raccords tubulaires 41 et 42 sont entourés par une jupe 45 et se prolongent par des éléments tubulaires 41A et 42A. La jupe 45 de l'embout 40 peut s'emboîter sur la jupe 14 de l'une quelconque des deux pièces d'extrémité 8. Lorsque la jupe 45 est emboîtée sur une jupe 14, les éléments tubulaires 41A et 42A pénètrent dans les passages traversants 10 et 11 correspondants, de sorte que les embouts 41 et 42 sont respectivement raccordés de façon étanche aux passages traversants 10 et 11 de la pièce 8 correspondante. L'embout 40 comporte des moyens de solidarisation par encliquetage 46 susceptibles de coopérer avec les moyens d'encliquetage 34 des pièces 8.

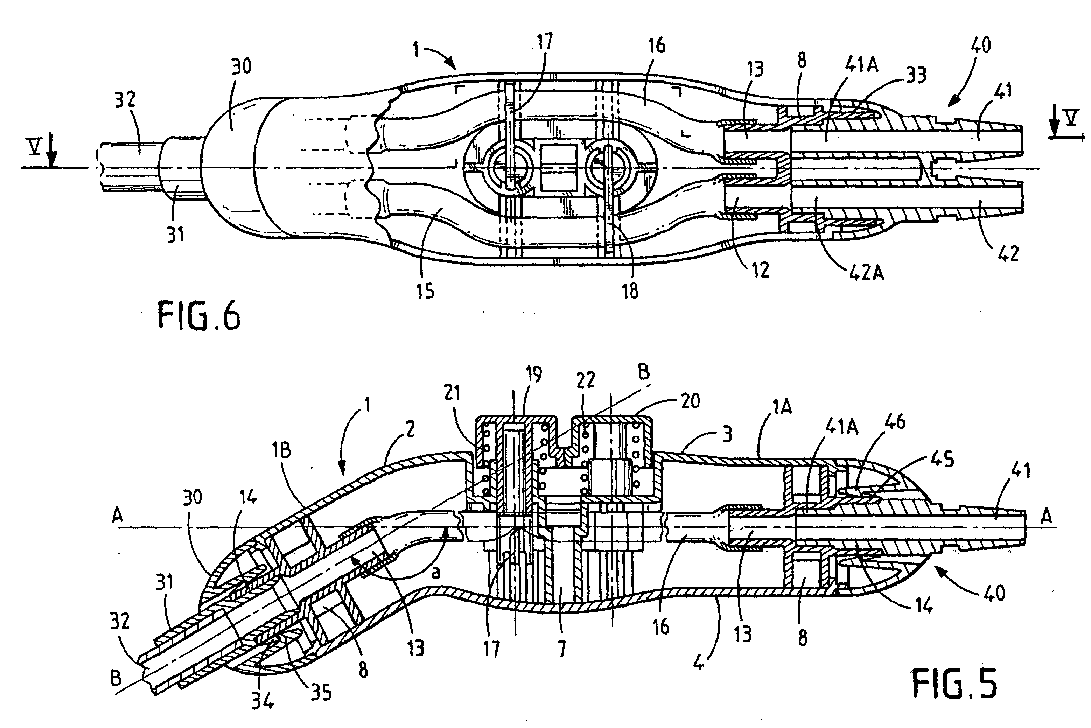

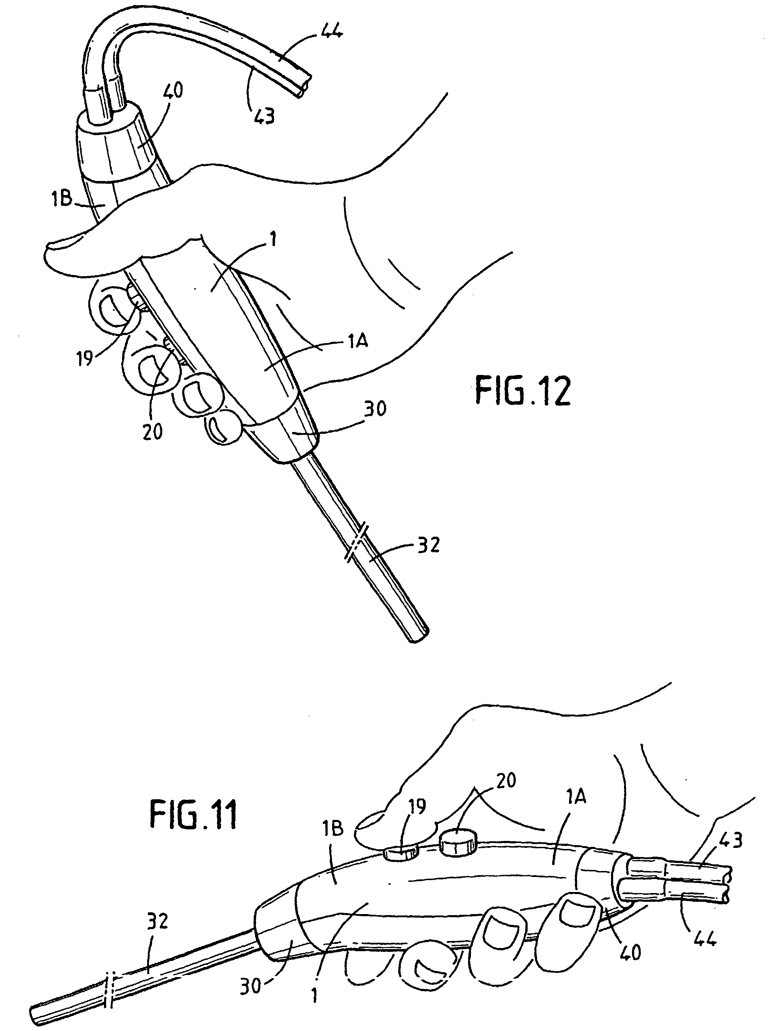

On comprend qu'ainsi les embouts 30 et 40 sont interchangeables et peuvent chacun être montés à l'une quelconque des extrémités du corps oblong 1, comme cela est illustré sur les figures 11 et 12. Sur les figures 5, 6 et 11, on a représenté l'embout 30 monté sur l'extrémité libre de la partie 1B du corps oblong 1, et l'embout 40 monté à l'extrémité libre de la partie 1A dudit corps 1. Au contraire, sur la figure 12, l'embout 30 est monté à l'extrémité libre de la partie 1A, tandis que l'embout 40 est monté à l'extrémité libre de la partie 1B du corps 1.

Les figures 11 et 12 montrent que, grâce à l'interchangeabilité des embouts 30 et 40 et au coude 2 du corps 1, l'appareil conforme à la présente invention peut être tenu de façons différentes, en fonction de la disposition de l'environnement et du confort du chirurgien.

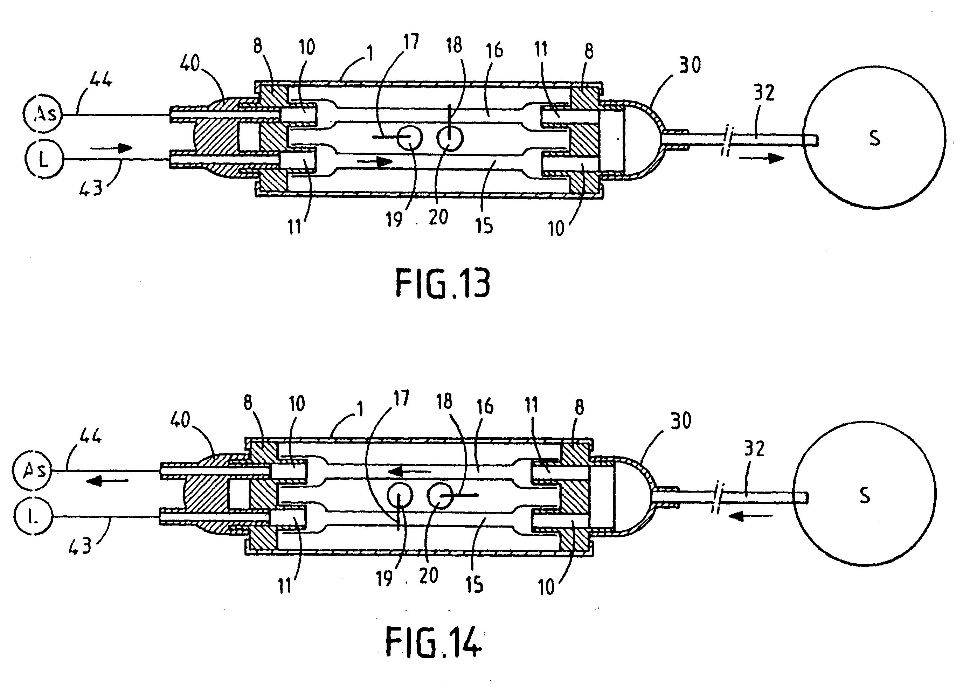

Sur les figures 13 et 14, on a illustré schématiquement un mode de fonctionnement de l'appareil conforme à la présente invention, en supposant que la conduite souple 15 est reliée à une source de liquide de lavage L par la conduite 43, tandis que la conduite souple 16 est reliée à un dispositif d'aspiration As par la conduite 44.

Ainsi, l'extrémité libre de la canule allongée 32 se trouvant en regard d'un site S à laver, si l'opérateur appuie sur le bouton-poussoir 19, du liquide de lavage passe de la source L au site S, à travers la conduite souple 15, la conduite souple 16 restant écrasée par l'action élastique de la guillotine 18 liée au bouton-poussoir 20 et isolant donc le dispositif d'aspiration As (voir la figure 13). Maintenant, si l'opérateur cesse d'appuyer sur le bouton-poussoir 19, mais appuie sur le bouton-poussoir 20, le liquide de lavage pollué est aspiré du site S par le dispositif As, à travers la conduite souple 16, la conduite souple 15 étant écrasée par l'action de la guillotine 17 liée au bouton-poussoir 19 et isolant donc la source de liquide de lavage L (voir la figure 14).

De ce qui précède, on comprendra aisément que le dispositif conforme à la présente invention peut être réalisé à bon marché, par exemple en matière synthétique. Il peut être présenté à la vente sous la forme d'un ensemble de plusieurs pièces séparées comprenant, par exemple, le corps oblong 1 complet, plusieurs embouts différents 30 et un embout 40. Le chirurgien choisit l'embout 30 qui convient à l'intervention à effectuer (longueur, diamètre, etc ... de la canule allongée 32), l'assemble à celle des extrémités du corps coudé 1 qui lui assure la prise la plus confortable et assemble l'embout 40 à l'autre extrémité.

Les moyens de solidarisation à encliquetage 34, 35, 46 peuvent être tels que, après un premier usage, le dispositif selon l'invention ne puisse plus être démonté.

## Revendications

1. Dispositif chirurgical pour réaliser des aspirations et des lavages de sites corporels (S), tels que des sites opératoires et des cavités corporelles, comportant :
- un corps oblong (1) destiné à servir de poignée pour un opérateur et coudé à l'une de ses extrémités ;
- un premier raccord tubulaire (31), disposé à une extrémité dudit corps oblong (1) et destiné à être mis en communication avec un tel site corporel (S), ledit premier raccord étant porté par un premier embout (30) pouvant se monter à ladite extrémité dudit corps oblong ;
- un deuxième et un troisième raccords tubulaires (41 et 42), disposés à l'autre extrémité dudit corps oblong (1)et destinés à être reliés, respectivement, à une source de liquide de lavage (L) et à des moyens d'aspiration (As) ; et
- des organes d'actionnement (19, 20) apparaissant à la surface dudit corps oblong (1) pour être à la disposition dudit opérateur,
**caractérisé en ce que** :
- à chacune de ses extrémités, ledit corps oblong comporte deux passages traversants (10, 11) respectivement reliés à deux lignes de liaison (15, 16) internes audit corps oblong (1), de sorte que chacune desdites lignes de liaison (15, 16) relie un passage traversant (10, 11) d'une extrémité dudit corps oblong à un passage traversant (10, 11) de l'autre extrémité dudit corps oblong ;
- des moyens de commande sélective (17, 18) sont montés dans ledit corps oblong (1) et sont susceptibles de commander la circulation fluide dans lesdites lignes de liaison (15, 16), lesdits moyens de commande sélective (17, 18) étant actionnés par lesdits organes d'actionnement (19, 20) qui se trouvent du côté dudit corps oblong (1) opposé à la concavité dudit coude (2) ;
- ledit premier embout (30) peut se monter à l'une quelconque des extrémités dudit corps oblong (1) de façon que ledit premier raccord tubulaire (31) soit raccordé, en commun et de façon étanche, aux deux passages traversant (10, 11) de l'extrémité sur laquelle est monté ledit premier embout (30) ; et
- il est prévu au moins un second embout (40), porteur desdits deuxième et troisième raccords tubulaires (41, 42) et pouvant se monter à l'une quelconque des extrémités dudit corps oblong (1), de façon que lesdits deuxième et troisième raccords tubulaires (41, 42) soient respectivement raccordés de façon étanche aux deux passages traversants (10, 11) de l'extrémité sur laquelle est monté ledit second embout (40).

2. Dispositif selon la revendication 1,
**caractérisé en ce que** les deux parties (1A et 1B) dudit corps oblong (1), séparées par ledit coude (2), sont sensiblement rectilignes.

3. Dispositif selon la revendication 2,
**caractérisé en ce que** l'angle (a) compris entre les axes (A-A et B-B) desdites parties (1A et 1B) dudit corps oblong (1) séparées par ledit coude (2) est au moins égal à 90°.

4. Dispositif selon la revendication 3,
**caractérisé en ce que** ledit angle (a) est au moins approximativement égal à 120°.

5. Dispositif selon l'une quelconque des revendications 1 à 4,
**caractérisé en ce que** ledit corps oblong (1) comporte, à chacune de ses extrémités, une jupe (14) entourant les deux passages traversants (10, 11) correspondants, lesdites jupes étant identiques et saillant vers l'extérieur, et **en ce que** chacun desdits premier et second embouts (30, 40) comporte également une jupe (33, 45) entourant respectivement ledit premier (31) et lesdits deuxième et troisième (41, 42) raccords tubulaires et susceptible de s'emboîter sur l'une desdites jupes (14) dudit corps oblong (1).

6. Dispositif selon la revendication 5,
**caractérisé en ce que** lesdites jupes (14) dudit corps oblong (1) et lesdites jupes (33, 45) desdits premier et second embouts (30, 40) comportent des moyens de verrouillage coopérants (34, 35, 46).

7. Dispositif selon l'une quelconque des revendications 5 ou 6,
**caractérisé en ce que** ledit second embout (40) comporte des prolongements (41A, 42A) respectifs desdits deuxième et troisième raccords tubulaires (41, 42), en saillie par rapport à la jupe (45) correspondante, lesdits prolongements (41A, 42A) étant susceptibles d'être introduits respectivement dans les deux passages traversants (10, 11) d'une extrémité dudit corps oblong (1).

8. Dispositif selon l'une quelconque des revendications 1 à 7,
**caractérisé en ce que** les deux lignes de liaison (15, 16) internes audit corps oblong (1) sont constituées par des conduites souples écrasables, **en ce que** lesdits moyens de commande sélective comprennent deux guillotines (17, 18) respectivement associées auxdites conduites souples et pressées par des moyens élastiques (21, 22) et **en ce que** lesdits organes d'actionnement sont des boutons-poussoirs (19, 20) solidaires desdites guillotines (17, 18).

9. Dispositif selon l'une quelconque des revendications 1 à 8,
**caractérisé en ce que** ledit corps oblong (1) comporte deux coques longitudinales coudées (3, 4) raccordées le long d'un plan de joint coudé (5) et deux pièces d'extrémité identiques (8), pourvues desdits passages traversants (10, 11) et emprisonnées entre lesdites coques (3, 4).

10. Dispositif selon la revendication 9,
**caractérisé en ce que** lesdites coques (3, 4), lesdites pièces d'extrémité (8) et lesdits premier et second embouts (30, 40) sont réalisés en matière synthétique.

11. Dispositif selon l'une des revendications 1 à 9,
**caractérisé en ce que** ledit premier raccord tubulaire (31) dudit premier embout (30) est solidaire d'une canule allongée (32).

12. Dispositif selon la revendication 11,
**caractérisé en ce qu'**il comporte au moins deux premiers embouts (30) interchangeables, pourvus de canules allongées (32) différentes.

## Patentansprüche

1. Chirurgische Vorrichtung zur Durchführung von Saug- und Spülvorgängen an Körperstellen (S), wie zum Beispiel Operationsstellen und Körperhohlräume, mit:
- einem länglichen Körper (1), der dazu bestimmt ist, einem Operateur als Handgriff zu dienen, und der an einem seiner Enden gekrümmt ist;
- einer ersten röhrenförmigen Verbindung (31), angeordnet an einem Ende des genannten länglichen Körpers (1) und dazu bestimmt, mit einer solchen Körperstelle (S) verbunden zu werden, wobei die genannte erste Verbindung durch ein erstes Ansatzstück (30) gehalten wird, das an das genannte Ende des genannten länglichen Körpers angebaut werden kann;
- einer zweiten und dritten röhrenförmigen Verbindung (41 und 42), angeordnet am anderen Ende des genannten länglichen Körpers (1) und dazu bestimmt, jeweils an eine Quelle zur Sputflüssigkeitsversorgung (L) und an Saugeinrichtungen (As) angeschlossen zu werden; und
- Betätigungselemente (19, 20), die auf der Oberfläche des genannten länglichen Körpers (1) erscheinen und damit dem genannten Operateur zur Verfügung stehen,
**dadurch gekennzeichnet, dass**
- der genannte längliche Körper an jedem Ende zwei Durchführungen (10, 11) besitzt, die jeweils mit zwei Verbindungsleitungen (15, 16) im Inneren des genannten länglichen Körpers (1) verbunden sind, so dass von jeder der genannten Verbindungsleitungen (15, 16) eine Durchführung (10, 11) an einem Ende des genannten länglichen Körpers mit einer Durchführung (10, 11) am anderen Ende des genannten länglichen Körpers verbunden wird;
- Mittel zur selektiven Steuerung (17, 18) in dem genannten länglichen Körper (1) eingebaut sind und in der Lage sind, den Flüssigkeitskreislauf in den genannten Verbindungsleitungen (15, 16) zu steuem, wobei die genannten Mittel zur selektiven Steuerung (17, 18) durch die genannten Betätigungselemente (19, 20) betätigt werden, die sich auf der Seite des genannten länglichen Körpers (1) gegenüber der Vertiefung des genannten gekrümmten Teils (2) befinden;
- das genannte erste Ansatzstück (30) an ein beliebiges Ende des genannten länglichen Körpers (1) so angebaut werden kann, dass die genannte erste röhrenförmige Verbindung (31) gemeinsam und dicht mit den beiden Durchführungen (10, 11) am Ende verbunden wird, an dem das genannte erste Ansatzstück (30) montiert ist; und
- zumindest ein zweites Ansatzstück (40) vorgesehen ist, das die genannte zweite und dritte röhrenförmige Verbindung (41, 42) trägt und an einem beliebigen Ende des genannten länglichen Körpers (1) so montiert werden kann, dass die genannte zweite und dritte röhrenförmige Verbindung (41, 42) jeweils dicht mit den beiden Durchführungen (10, 11) an dem Ende verbunden wird, an dem das genannte zweite Ansatzstück (40) angebaut wird.

2. Vorrichtung nach Anspruch 1,
**dadurch gekennzeichnet, dass** die beiden Teile (1A und 1B) des genannten länglichen Körpers (1), die durch den genannten gekrümmten Teil (2) getrennt werden, im Wesentlichen geradlinig sind.

3. Vorrichtung nach Anspruch 2,
**dadurch gekennzeichnet, dass** der Winkel (a) zwischen den Achsen (A-A und B-B) der genannten Teile (1A und 1B) des genannten länglichen Körpers (1), die durch den genannten gekrümmten Teil (2) getrennt sind, mindestens 90° beträgt.

4. Vorrichtung nach Anspruch 3,
**dadurch gekennzeichnet, dass** der genannte Winkel (a) mindestens ca. 120° beträgt.

5. Vorrichtung nach einem der Ansprüche 1 bis 4,
**dadurch gekennzeichnet, dass** der genannte längliche Körper (1) an jedem Ende mit einer Ummantelung (14) versehen ist, von der die beiden entsprechenden Durchführungen (10, 11) umgeben werden, wobei die genannten Ummantelungen identisch sind und nach außen hervorspringen, und dass sowohl das genannte erste als auch das genannte zweite Ansatzstück (30, 40) ebenfalls eine Ummantelung (33, 45) umfasst, die jeweils die genannte erste röhrenförmige Verbindung (31) und die genannte zweite und dritte röhrenförmige Verbindung (41, 42) umgibt und in der Lage ist, auf eine der genannten Ummantelungen (14) des genannten länglichen Körpers (1) gesteckt werden zu können.

6. Vorrichtung nach Anspruch 5,
**dadurch gekennzeichnet, dass** die genannten Ummantelungen (14) des genannten länglichen Körpers (1) und die genannten Ummantelungen (33, 45) des genannten ersten und zweiten Ansatzstückes (30, 40) mit zusammenwirkenden Verriegelungsmitteln (34, 35, 46) versehen sind.

7. Vorrichtung nach einem der Ansprüche 5 bzw. 6,
**dadurch gekennzeichnet, dass** das genannte zweite Ansatzstück (40) mit jeweiligen Verlängerungen (41A, 42A) der genannten zweiten und dritten röhrenförmigen Verbindung (41, 42) versehen ist, die im Vergleich zur entsprechenden Ummantelung (45) hervorspringen, wobei die genannten Verlängerungen (41A, 42A) jeweils in die beiden Durchführungen (10, 11) an einem Ende des genannten länglichen Körpers (1) eingeführt werden können.

8. Vorrichtung nach einem der Ansprüche 1 bis 7,
**dadurch gekennzeichnet, dass** die beiden Verbindungsleitungen (15, 16) im Inneren des genannten länglichen Körpers (1) aus flexiblen zusammendrückbaren Leitungen bestehen, dass die genannten Mittel zur selektiven Steuerung mit zwei Quetschvorrichtungen (17, 18) versehen sind, die jeweils mit den genannten flexiblen Leitungen verbunden sind und durch elastische Mittel (21, 22) zusammengedrückt werden, und dass es sich bei den genannten Betätigungsetementen um Druckknöpfe (19, 20) handelt, die mit den genannten Quetschvorrichtungen (17, 18) fest verbunden sind.

9. Vorrichtung nach einem der Ansprüche 1 bis 8,
**dadurch gekennzeichnet, dass** der genannte längliche Körper (1) zwei gekrümmte Längsschalen (3, 4) umfasst, die entlang einer gekrümmten Stoßebene (5) miteinander verbunden sind, sowie zwei identische Endstücke (8), die mit den genannten Durchführungen (10, 11) versehen sind und zwischen den genannten Schalen (3, 4) eingeschlossen sind.

10. Vorrichtung nach Anspruch 9,
**dadurch gekennzeichnet, dass** die genannten Schalen (3, 4), die genannten Endstücke (8) und das genannte erste und zweite Ansatzstück (30, 40) aus synthetischem Material hergestellt werden.

11. Vorrichtung nach einem der Ansprüche 1 bis 9,
**dadurch gekennzeichnet**, die genannte erste röhrenförmige Verbindung (31) des genannten ersten Ansatzstückes (30) fest mit einer verlängerten Kanüle (32) verbunden ist.

12. Vorrichtung nach Anspruch 11,
**dadurch gekennzeichnet, dass** diese Vorrichtung mindestens zwei erste austauschbare Ansatzstücke (30) umfasst, die mit verschiedenen verlängerten Kanülen (32) ausgestattet sind.

## Claims

1. Surgical device for performing aspiration and irrigation of body sites (S), such as operating sites and body cavities, including:
- an oblong body (1) intended to serve as a grip for an operator and curved at one of its ends;
- a first tubular connector (31) arranged at one end of the said oblong body (1) and intended to be brought into communication with such a body site (S), the said first connector being carried by a first joining piece (30) capable of being mounted at the said end of the said oblong body;
- second and third tubular connectors (41 and 42) arranged at the other end of the said oblong body (1) and intended to be connected, respectively, to a source of irrigating liquid (L) and to aspiration means (As); and
- actuating members (19, 20) appearing at the surface of the said oblong body (1) so as to be available to the said operator,
**characterized in that**:
- at each of its ends, the said oblong body includes two through passages (10, 11) connected respectively to two connecting lines (15, 16) inside the said oblong body (1), in such a way that each of the said connecting lines (15, 16) connects one through passage (10, 11) at one end of the said oblong body to a through passage (10, 11) at the other end of the said oblong body;
- selective control means (17, 18) are mounted in the said oblong body (1) and are capable of controlling the fluid circulation in the said connecting lines (15, 16), the said selective control means (17, 18) being actuated by the said actuating members (19, 20) which are located on that side of the said oblong body (1) opposite the concavity of the said curve (2);
- the said first joining piece (30) is capable of being mounted at either one of the ends of the said oblong body (1), in such a way that the said first tubular connector (31) is connected, in common and in a sealed manner, to the two through passages (10, 11) of the end on which the said first joining piece (30) is mounted; and
- there is at least one second joining piece (40) carrying the said second and third tubular connectors (41, 42) and capable of being mounted at either one of the ends of the said oblong body (1), in such a way that the said second and third tubular connectors (41, 42) are connected respectively in a sealed manner to the two through passages (10, 11) of the end on which the said second joining piece (40) is mounted.

2. Device according to Claim 1, **characterized in that** the two parts (1A and 1B) of the said oblong body (1), separated by the said curve (2), are substantially rectilinear.

3. Device according to Claim 2, **characterized in that** the angle (a) between the axes (A-A and B-B) of the said parts (1A and 1B) of the said oblong body (1) separated by the said curve (2) is at least equal to 90°.

4. Device according to Claim 3, **characterized in that** the said angle (a) is at least approximately equal to 120°.

5. Device according to any one of Claims 1 to 4, **characterized in that** the said oblong body (1) includes, at each of its ends, a skirt (14) surrounding the two corresponding through passages (10, 11), the said skirts being identical and projecting outward, and **in that** each of the said first and second joining pieces (30, 40) also includes a skirt (33, 45) surrounding respectively the said first (31) and the said second and third (41, 42) tubular connectors and being capable of engaging on one of the said skirts (14) of the said oblong body (1).

6. Device according to Claim 5, **characterized in that** the said skirts (14) of the said oblong body (1) and the said skirts (33, 45) of the said first and second joining pieces (30, 40) include cooperating locking means (34, 35, 46).

7. Device according to either of Claims 5 or 6, charaterized in that the said second joining piece (40) includes respective continuations (41A, 42A) of the said second and third tubular connectors (41, 42), projecting relative to the corresponding skirt (45), the said continuations (41A, 42A) being capable of being introduced respectively into the two through passages (10, 11) of one end of the said oblong body (1).

8. Device according to any one of Claims 1 to 7, **characterized in that** the two connecting lines (15, 16) inside the said oblong body (1) are made up of flexible conduits which are crushable, **in that** the said selective control means comprise two guillotines (17, 18) respectively associated with the said flexible conduits and pressed by elastic means (21, 22), and **in that** the said actuating members are push buttons (19, 20) integral with the said guillotines (17, 18).

9. Device according to any one of Claims 1 to 8, **characterized in that** the said oblong body (1) includes two curved longitudinal shells (3, 4) which are connected along a curved joining plane (5) and two identical end components (8) which are provided with the said through passages (10, 11) and are held between the said shells (3, 4).

10. Device according to Claim 9, **characterized in that** the said shells (3, 4), the said end components (8) and the said first and second joining pieces (30, 40) are made of synthetic material.

11. Device according to one of Claims 1 to 9, **characterized in that** the said first tubular connector (31) of the said first joining piece (30) is integral with an elongate cannula (32).

12. Device according to Claim 11, **characterized in that** it includes at least two interchangeable first joining pieces (30) which are provided with different elongate cannulas (32).
